# EUROPEAN PATENT APPLICATION

(11) **EP 4 437 997 A1**
(43) Date of publication of application: **02.10.2024**
(21) Application number: 22930609.7
(22) Date of filing: 18.11.2022
(51) Int. Cl.: A61B 34/37

(54) **DOCTOR-CONTROLLED PLATFORM BASE**

(30) Priority: 09.03.2022 CN 202210223678
(71) Applicant: Agibot Medtech (Suzhou) Co., Ltd., Suzhou, Jiangsu 215123 (CN)
(72) Inventor: XI, Xueping, Suzhou, Jiangsu 215123 (CN); PENG, Cheng, Suzhou, Jiangsu 215123 (CN); XU, Min, Suzhou, Jiangsu 215123 (CN)
(74) Representative: Algemeen Octrooi- en Merkenbureau B.V.
(86) International application number: PCT/CN2022/132768
(87) International publication number: WO 2023/168986

(57) **Abstract**

A doctor-controlled platform base, comprising a base (1), synchronous pedal assemblies, a control pedal assembly and a brake assembly; the synchronous pedal assemblies are arranged at the bottoms of two sides of the base (1); two sides of the control pedal assembly are connected to the lower surface of the base (1), and the two sides of the control pedal assembly are connected to the synchronous pedal assemblies; a walking device is arranged below the base (1); one end of the brake assembly is hingedly connected to the control pedal assembly, and the other end of the brake assembly is connected to the walking device; when the at least one synchronous pedal assembly is subjected to an external force, the two synchronous pedal assemblies ascend and descend synchronously, and simultaneously drive the control pedal assembly to ascend and descend in the same direction; when ascending and descending, the control pedal assembly can drive the brake assembly to move to lock or loosen the walking device. According to the device, by stepping down synchronous pedals (2), the control pedal assembly can ascend or descend synchronously, thereby simplifying the driving mechanism, saving the cost, and improving the use comfort; the synchronous pedal assemblies can be stabilized at the highest stable point or the lowest stable point.

## Description

This application claims priority to Chinese Patent Application No. 202210223678.0, titled "doctor-controlled platform base", filed on 9 March 2022 with the China National Intellectual Property Administration (CNIPA), which is incorporated herein by reference in its entirety.

### FIELD

The present disclosure relates to the field of medical equipment, and more particularly, to a doctor-controlled platform base.

### BACKGROUND

With the development of technology, minimally invasive surgery has been more and more widely used because of its advantages such as less surgical trauma, short recovery time and less pain for patients. Laparoscopic surgical robots are also developing rapidly with the promotion of minimally invasive surgery. It is a new minimally invasive surgical device, which is composed of a doctor-controlled platform, a surgery platform for patient and an image platform. An upper end of the doctor-controlled platform is equipped with a master mechanical arm convenient for hand operation, and a lower end of the doctor-controlled platform is equipped with a pedal convenient for foot operation. Surgeons operate the master mechanical arm on the doctor-controlled platform by hand. Doctors remotely control a surgical mechanical arm at the surgery platform for patient through operating the master mechanical arm by hands and operating the control pedal (or button, etc.) by foot, such that the surgical mechanical arm perform incision, separation, suture and many other complex actions on patient's body to complete the operation.

The doctor-controlled platform may be deployed by manual pushing. A height difference between universal casters of the platform and the lowest point of the chassis of the platform is needed so as to ensure that the doctor-controlled platform is capable of passing on certain terrains when the doctor-controlled platform is pushed. After the doctor-controlled platform is deployed, the universal casters need to be locked to ensure a stable operating platform. In addition, pedal bases which are provided with various control pedal switches need to fall down to the ground to allow the surgeon to operate with his foot more comfortably.

Two control mechanisms are needed for controlling the locking of the existing doctor-controlled platform and the falling of the pedal base, respectively, which leads to complexity of the whole equipment. In some cases, there is only a locking mechanism for the doctor-controlled platform, without an up-down mechanism for the pedal, and thus the pedal is maintained at a high-level position, causing the leg and foot of the surgeon being suspended from the ground when the surgeon operates the control pedal switches, which is not comfortable for the surgeon to operate the pedal. For example, Patent CN207870961U proposes a doctor-controlled board of a surgical robot which has a simple structure, can effectively improve operating comfort and accuracy of the doctor. The doctor-controlled board includes an operating platform, an up-down post is provided at a bottom of the operating platform, with a top portion of the up-down post fixedly connected to the operating platform and a bottom portion of the up-down post fixedly connected with a base frame. The up-down post is provided with an outer shell, and the outer shell moves up or down synchronously with the operating platform. By connecting the operating platform of the doctor-controlled board with the up-down post, the height of the operating platform can be adjusted and thus the doctor can perform the operation more flexibly, which improves the practicability of the equipment, and effectively improves the operating accuracy and comfort of the doctor. However, the doctor-controlled board does not involve simplifications of caster-locking mechanism at the bottom and the up-down mechanism for the pedal.

### SUMMARY

In order to solve the above problems, this disclosure provides a doctor-controlled platform base, aiming to simplify up-down mechanism for a base pedal and to improve the usage comfort.

To achieve the above purposes, this disclosure provides the following technical solutions.

A doctor-controlled platform base includes a U-shaped base, synchronous pedal assemblies, a control pedal assembly and a brake assembly; bottoms of two sides of the U-shaped base are provided with the synchronous pedal assemblies through first balance assemblies and first guide mechanisms;
the control pedal assembly is arranged in an opening of the U-shaped base, and two sides of the control pedal assembly are connected with a lower surface of the U-shaped base through second balance assemblies and second guide mechanisms, and the two sides of the control pedal assembly are connected with the synchronous pedal assemblies;
a walking device is arranged under the U-shaped base, one end of the brake assembly is connected with the control pedal assembly in a hinged manner and the other end of the brake assembly is connected with the walking device; and
two synchronous pedal assemblies move up or down synchronously if an external force is applied to at least one of the synchronous pedal assemblies, and simultaneously drive the control pedal assembly to move up or down in the same direction as the synchronous pedal assemblies; the control pedal assembly, when moving up or down, can drive the brake assembly to move, to realize locking or loosening of the walking device.

Further, the control pedal assembly includes a control pedal support, a control pedal mounting seat, the second balance assemblies and the second guide mechanisms, the control pedal mounting seat is arranged on the control pedal support, one end of the second balance assembly is connected with the control pedal support, and the other end of the second balance assembly is connected with the U-shaped base; the control pedal support is connected with the synchronous pedal assemblies.

Further, one of the control pedal support and the synchronous pedal assembly is provided with a convex snap fitting feature, and the other of the control pedal support and the synchronous pedal assembly is provided with a concave snap fitting feature capable of matching with the convex snap fitting feature.

The convex snap fitting feature is a synchronous pedal-hanging pillar and the concave snap fitting feature is an U-shaped groove, the synchronous pedal-hanging pillar includes a cylindrical support rod and a baffle plate arranged at an upper end of the support rod, and the U-shaped groove is clamped on the support rod.

Further, the control pedal support is connected with the synchronous pedal assembly in a hinged manner or in a snap fit manner, and in case that they are connected in a hinged manner, the control pedal support is provided thereon with a hinged bar, and the synchronous pedal assembly is hingedly connected with the hinged bar.

Further, the second guide mechanism includes two second guide shafts and two second guide sleeves, the second guide shafts are arranged at two sides of the second balance assembly, each second guide shaft is inserted in one second guide sleeve, and one end of the second guide sleeve is fixedly connected with the U-shaped base; and the second guide shaft slides in the second guide sleeve.

Further, one side of the control pedal mounting seat closing to a closed end of the U-shaped base is connected with the control pedal support through a telescopic device.

Further, the telescopic device includes a leading screw fixing sleeve, a leading screw, a driven gear, a driving gear and a driving mechanism, the leading screw fixing sleeve is disposed at a bottom of the control pedal mounting seat, the leading screw is arranged in the leading screw fixing sleeve, the driven gear is arranged at the other end of the leading screw, and the driven gear is engaged with the driving gear; the driven gear and the driving gear are both arranged on the control pedal support, and the driving mechanism drives the driving gear to rotate; the driven gear is connected with the leading screw through threads, and the control pedal support is provided therein with a through hole corresponding to the leading screw, and the leading screw is movable in the through hole in a telescopic manner.

Further, the control pedal support includes a main support, link receiving plates and second mounting plates, the main support is arranged under the closed end of the U-shaped base, the link receiving plates are arranged at two sides of the main support, and the second mounting plates are arranged at outer sides of the link receiving plates; two sides of the control pedal mounting seat are provided with guide rails, one side of each link receiving plate is provided with a slide block, and the control pedal mounting seat is slidably connected with the link receiving plates through the guide rails and slide blocks.

Further, the synchronous pedal assemblies include two synchronous pedals, two first mounting plates, two first balance assemblies and two first guide mechanisms; the two first mounting plates are arranged on two sides of the U-shaped base, respectively, and the synchronous pedals and the first balance assemblies are arranged on the first mounting plates; the first balance assemblies are fixed under the U-shaped base, and the synchronous pedals are connected with the control pedal assembly.

Further, the first guide mechanism includes two first guide shafts and two first guide sleeves, the first guide shafts are arranged at two sides of the first balance assembly, each first guide shaft is inserted in one first guide sleeve, and one end of the first guide sleeve is fixedly connected with the U-shaped base; the first guide shaft slides in the first guide sleeve.

Further, the first balance assembly includes a mobile mounting seat, an elastic component and a fixed mounting seat, the fixed mounting seat is fixedly connected to the U-shaped base under the U-shaped base, and the mobile mounting seat is disposed on the first mounting plate; one end of the elastic component is connected with the fixed mounting seat, and the other end of the elastic component is connected with the mobile mounting seat.

Further, the elastic component is a spring, a bellow, a diaphragm or other elastic component having a certain resilience.

Further, the synchronous pedal assembly includes a synchronous shaft and two synchronous pedal links; ends of the synchronous shaft are fixedly connected with the synchronous pedal links, and each synchronous pedal link is hingedly connected with a corresponding first mounting plate.

Further, the synchronous pedal includes a pedal plate provided with a limit structure, and a limit shaft is mounted on the U-shaped base; the limit shaft is inserted in the limit structure; the limit structure has at least an upper limit part and a lower limit part; the limit structure is capable of coordinating with the upper limit part or the lower limit part to achieve positioning upon the synchronous pedal assembly moves up or down to a preset position.

Further, the limit structure includes a rotation shaft and a limit groove sleeve, the rotation shaft is inserted in the limit groove sleeve, a limit groove is provided in an outer wall of the limit groove sleeve, a distance between the limit groove and a bottom surface of the pedal plate increases or decreases with rotation of the limit groove sleeve; the limit shaft is inserted in the limit groove.

Further, the doctor-controlled platform base further includes a sensing device for determining position of the synchronous pedal; the sensing device includes a sensing plate and a proximity switch; the sensing plate or the proximity switch is installed on the U-shaped base.

Further, the walking device includes at least two casters with central control brake; the brake assembly includes at least two brake shaft seats, brake shafts and brake links;
the brake shaft seats are arranged under two sides of the open end of the U-shaped base and are close to the casters with central control brake, one end of the brake link is hingedly connected with the control pedal assembly, and the other end of the brake link is connected with the caster with central control brake through the brake shaft;
the brake shaft is inserted in the brake shaft seat, and is rotatable in the brake shaft seat so as to realize locking or loosening of a driving wheel.

Further, the first guide mechanism has the same structure as the second guide mechanism.

Compared with the prior art, this disclosure has the following beneficial effects.
(1) In this disclosure, synchronous pedal assemblies are arranged under the U-shaped base, and the synchronous pedal assemblies are fixedly connected to the U-shaped base through the first balance assemblies, and the first balance assemblies provide upward pulling forces to the synchronous pedals. The control pedal assembly is fixedly connected to the U-shaped base through the second balance assemblies, the second balance assemblies provide upward pulling forces to the control pedal mounting seat, meanwhile, the synchronous pedals are connected to the control pedal assembly through the synchronous pedal-hanging pillars, and thus, when the synchronous pedals are pressed down, the synchronous pedals will drive the control pedal mounting seat to move downwards. When the synchronous pedals are at a lowest point, the control pedal mounting seat is also at a lowest position, which is convenient for a doctor to use the pedal or button on the control pedal mounting seat, improving the usage comfort. When the synchronous pedals move upwards, the synchronous pedals drive the control pedal mounting seat to move upwards through the synchronous pedal-hanging pillars. When the synchronous pedals rise to a highest point, the control pedal mounting seat is also at a highest position. In this way, linkage movement between the synchronous pedals and the control pedal mounting seat is realized.
(2) In this disclosure, the synchronous pedal assemblies include two first mounting plates arranged on two sides of the U-shaped base, respectively. Each first mounting plate is provided with a first balance assembly. The two first mounting plates are connected via a synchronous shaft, and the synchronous shaft is hingedly connected with the first mounting plates through synchronous pedal links. When one synchronous pedal at one side is pressed downwards, the first mounting plate at the same side is also moved downwards, the synchronous pedal link drives the synchronous shaft to rotate, and the rotation of the synchronous shaft drives a first mounting plate on the other side to move down, such that synchronous pedals at both sides are moved down at the same time when only one synchronous pedal at one side is pressed down. Therefore, movements of two sides of the doctor-controlled platform base are consistent, and the doctor-controlled platform base is more stable and easy to use.
(3) In this disclosure, the synchronous pedal includes a pedal plate which is a hollow square piece. A rotation shaft is arranged in the pedal plate, the rotation shaft is inserted in a limit groove sleeve. A limit groove is provided in the outer wall of the limit groove sleeve. In addition, a limit shaft fixing seat is arranged on the U-shaped base, and a limit shaft is disposed on the limit shaft fixing seat and is inserted into the limit groove. The limit groove is designed to have an increased or decreased distance from the bottom surface of the pedal plate with the rotation of the limit groove sleeve. Therefore, when the pedal plate is stepped downwards, the limit groove sleeve rotates due to the limitation of the limit shaft, and the limit shaft moves in the limit groove, the limit groove has a highest stable point and a lowest stable point, and thus the limit shaft can be stabilized at the highest stable point or the lowest stable point, thereby the pedal plate can stop stably in a highest or lowest position.
(4) In this disclosure, two end portions of the link receiving plate are hingedly connected with the brake links, the brake link near the synchronous shaft is fixed on the rear brake synchronous shaft seat, and the brake link away from the synchronous shaft is fixed on the front brake shaft seat. The brake link is connected to the rear brake synchronous shaft seat or the front brake shaft seat through the brake shaft, and the rotation of the brake link drives the brake shaft to rotate, and the rotation of the brake shaft can lock or loosen the caster. When the synchronous pedal is stepped downwards, the synchronous pedal drives the control pedal assembly to move downwards, and downwards movement of the control pedal assembly drives the brake shaft to rotate through the brake link, to achieve locking of the caster. Similarly, when the synchronous pedal is stepped downwards again, the synchronous pedal rises under pulling force of the first balance assembly, and at the same time, the control pedal assembly rises, and the brake link drives the brake shaft to rotate in an opposite direction to loosen the caster. That is, the caster can be locked or loosened by stepping on the synchronous pedal, and the pedal assemblies can be controlled to move up or down simultaneously, thereby simplifying the driving mechanism and improving the usage comfort.

### BRIEF DESCRIPTION OF THE DRAWINGS

Figure 1 schematically shows a structure according to the present disclosure.
Figure 2 schematically shows a structure according to the present disclosure, with the U-shaped base being hidden.
Figure 3 schematically shows a structure according to the present disclosure, after being turned at 180°, with synchronous pedal assemblies and the control pedal assembly being hidden.
Figure 4 is a structural schematic diagram illustrating the first balance assembly according to the present disclosure.
Figure 5 is a structural schematic diagram illustrating the control pedal assembly according to the present disclosure.
Figure 6 is a structural schematic diagram illustrating the synchronous pedal assemblies according to the present disclosure.
Figure 7 is a structural schematic diagram illustrating the brake link and the brake shaft according to the present disclosure.
Figure 8 is a structural schematic diagram illustrating the synchronous pedal according to the present disclosure.
Figure 9 is a deployment schematic diagram illustrating a limit groove according to the present disclosure.

Reference signs shown in the drawings:
1- U-shaped base, 2-synchronous pedal, 201-pedal plate, 202-rotation shaft, 203- limit groove sleeve, 3-first mounting plate, 4-first balance assembly, 401-mobile mounting seat, 402-elastic component, 403-fixed mounting seat, 5-first guide shaft, 6-rear brake synchronous shaft seat, 7-synchronous pedal link, 8-synchronous shaft, 9-proximity switch, 10-control pedal support, 1001-main support, 1002-link receiving plate, 1003-second mounting plate, 11-synchronous pedal-hanging pillar, 12-brake link, 13-brake shaft, 14-hinged link, 15-hinged shaft, 16-control pedal mounting seat, 17-second balance assembly, 18-leading screw fixing sleeve, 19-leading screw, 20-driven gear, 21-driving gear, 22-second guide shaft, 23-guide rail; 24-caster, 25-front brake shaft seat, 26-first guide sleeve, 27-second guide sleeve, 28-limit shaft fixing seat, 29-limit shaft, 30-synchronous pedal sensing plate; 31-high stable stop point, 32- high-low transition unstable stop point, 33-low stable stop point, 34- low-high transition unstable stop point.

### DETAILED DESCRIPTION OF EMBODIMENTS

Herein, the technical solutions of this disclosure will be described in detail in combination with the accompany drawings. Obviously, the embodiments described herein are not all of the embodiments of the present application, and all other embodiments obtained by those skilled in the art without making creative labor fall within the protection scope of the present application. It should be noted that the orientation or position relationships indicated by the terms "center", "up", "down", "left", "right", "vertical", "horizontal", etc., are based on the orientation or position relationships illustrated in the drawings and are only for facilitating the description of this application and simplifying the description, rather than indicating or implying that the device or element referred to must have the specific orientation, be configured and operated in the specific orientation, and therefore it cannot be construed as a limitation to this disclosure.

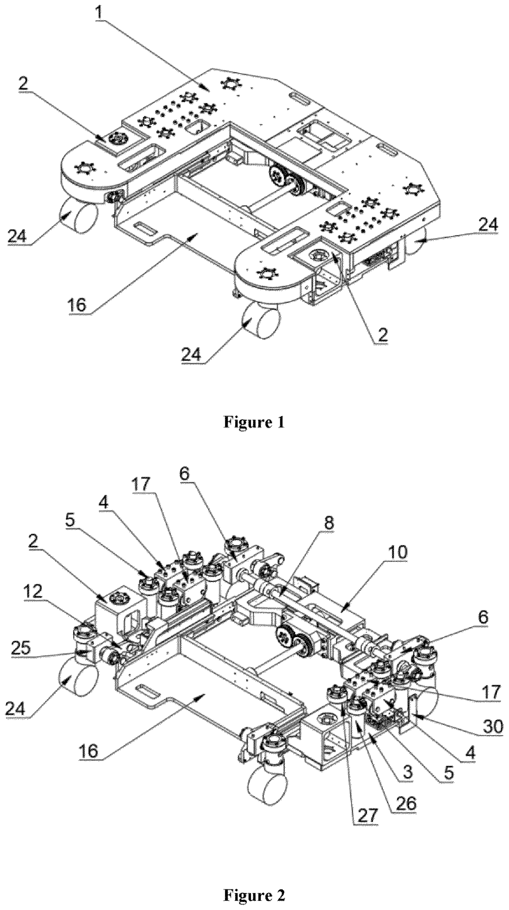

As shown in Figure 1, the present application provides a doctor-controlled platform base including a U-shaped base 1, synchronous pedal assemblies, a control pedal assembly and a brake assembly. The synchronous pedal assemblies are disposed at bottoms of two sides of the U-shaped base 1 through first balance assemblies 4 and first guide mechanisms. The control pedal assembly is arranged in an opening of the U-shaped base 1, and two sides of the control pedal assembly are connected to a lower surface of the U-shaped base 1 through second balance assemblies 17 and second guide mechanisms, and are connected to the synchronous pedal assemblies.

A walking device is arranged under the U-shaped base 1. One end of the brake assembly is hingedly connected with the control pedal assembly, and the other end of the brake assembly is connected with the walking device. There are two synchronous pedal assemblies, which move up or down synchronously when at least one of the synchronous pedal assemblies is subjected to an external force, which synchronously drive the control pedal assembly to move up or down in the same direction. The control pedal assembly, when moving up or down, can drive the brake assembly to move, to realize locking or loosening of the walking device.

The synchronous pedal assembly includes a synchronous pedal 2, a first mounting plate 3 and a first balance assembly 4. The synchronous pedal 2 and the first balance assembly 4 are arranged on the first mounting plate 3. The first balance assembly 4 is fixed under the U-shaped base 1. The control pedal assembly includes a control pedal support 10, a control pedal mounting seat 16 and second balance assemblies 17. The control pedal mounting seat 16 and the second balance assemblies 17 are arranged on the control pedal support 10. The second balance assemblies 17 are fixed under the U-shaped base 1.

The control pedal support 10 is connected with the synchronous pedal assemblies in a hinged or snap fit manner. In this embodiment, the control pedal support 10 is connected with the synchronous pedal assemblies in a snap fit manner. One of the control pedal support 10 and the synchronous pedal assembly is provided with a convex snap fitting feature, and the other of the control pedal support 10 and the synchronous pedal assembly is provided with a concave snap fitting feature capable of matching with the convex snap fitting feature.

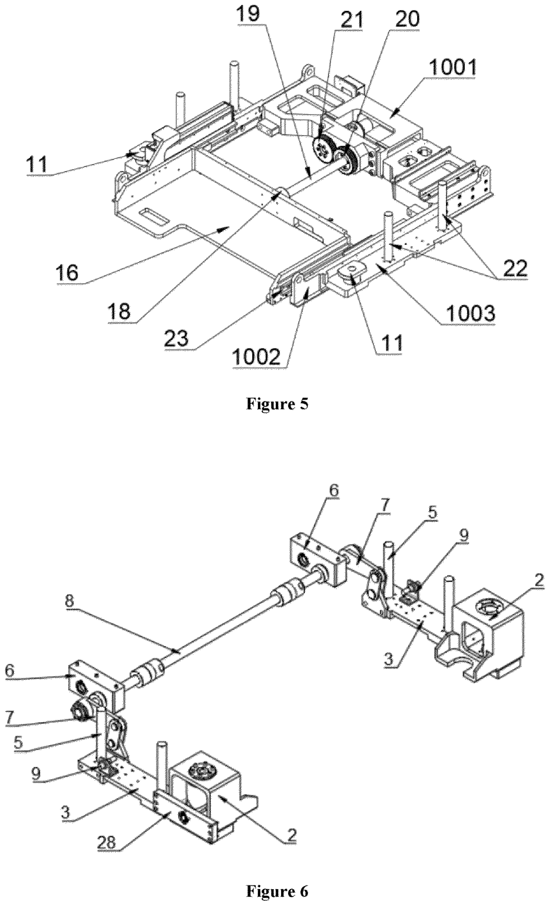

Preferably, the convex snap fitting feature is a synchronous pedal-hanging pillar 11, and the concave snap fitting feature is an U-shaped groove. The synchronous pedal-hanging pillar 11 is arranged on the control pedal support 10, and the U-shaped groove is arranged on the synchronous pedal assembly. As shown in Figure 5, the synchronous pedal-hanging pillar 11 includes a cylindrical support rod and a baffle plate arranged at an upper end of the support rod. As shown in Figure 6, the synchronous pedal 2 includes a pedal plate 201, an extension plate is arranged at one side of the pedal plate 201, and the U-shaped groove is provided in the extension plate. The U-shaped groove can be clamped on the support rod, so as to achieve linkage control of the synchronous pedal 2 and the control pedal mounting seat 16.

In other embodiments, the synchronous pedal-hanging pillar 11 may be arranged on the synchronous pedal assembly, and the U-shaped groove may be arranged on the control pedal assembly.

In other embodiments, the convex snap fitting feature may be a hook, and the concave snap fitting feature may be a ring. The hook is provided with a gap which facilitates to hang the hook into the ring. The hook may have a variety of shapes such as square, round and U-shape, which is not limited herein. The hook may be arranged on one of the control pedal assembly and the synchronous pedal assembly, and the ring is arranged on the other of the control pedal assembly and the synchronous pedal assembly.

In other embodiments, the control pedal support may be hingedly connected with the synchronous pedal assembly. The control pedal support 10 is provided thereon with a hinged bar. One end of the hinged bar is fixed on the control pedal support 10, and the other end of the hinged bar is provided with a ring. A post is arranged on the synchronous pedal 2, and the post can be placed in the ring of the hinged bar such that the post is hingedly connected with the hinged bar. An upper end of the post is provided with a limit flange, and the hinged bar is movable up and down a distance on the post.

The U-shaped base 1 is U-shaped. The synchronous pedal assemblies are arranged on the periphery portion of the U-shaped base 1, and the control pedal assembly is arranged in the middle portion of the U-shaped base 1. The control pedal mounting seat 16 is arranged in the internal hollow area of the U-shaped base 1. The U-shaped base 1 is the main frame of the doctor-controlled platform base, and the synchronous pedal assemblies and the control pedal assembly are all installed on the U-shaped base 1. The first balance assemblies, on the one hand, are configured to balance the weights of the synchronous pedal assemblies and to provide power for up-down movement of the synchronous pedal assemblies, and on the other hand, are configured to provide power for the control pedal assembly through the synchronous pedal-hanging pillars 11. The second balance assemblies 17 provide power for up-down movement of the control pedal assembly. When the synchronous pedal 2 is moved up or down under action of an external force, the synchronous pedal 2 drives the control pedal support 10 to move up or down through the synchronous pedal-hanging pillars 11, and the control pedal support 10 in turn drives the control pedal mounting seat 16 to move up or down, realizing synchronous up or down movements of the synchronous pedals 2 and the control pedal. Various control pedal switches are arranged on the control pedal mounting seat 16, so as to control certain actions of the surgical robot through the foot.

As shown in Figure 2, there are two first mounting plates 3, which are arranged on the two sides of the U-shaped base 1, respectively. As shown in Figure 6, the synchronous pedal assemblies also include two rear brake synchronous shaft seats 6 and synchronous pedal links 7. The rear brake synchronous shaft seats 6 are mounted on the U-shaped base 1, and the two rear brake synchronous shaft seats 6 are connected through a synchronous shaft 8. The synchronous shaft 8 passes through the rear brake synchronous shaft seats 6 and extends outwards, and two ends of the synchronous shaft 8 are fixedly connected with the synchronous pedal links 7. Preferably, the synchronous pedal link 7 hold the end of the synchronous shaft 8 through an expansion sleeve. The synchronous pedal link 7 is hingedly connected with the first mounting plate 3. One side of the first mounting plate 3 is provided with an upwardly raised triangle plate, and the synchronous pedal link 7 is hingedly connected with the triangle plate through two small plates and a pin shaft. The synchronous pedal assemblies are arranged symmetrically on the two sides of the U-shaped base 1. When an external force is applied to the synchronous pedal assembly on one side, the synchronous pedal on the one side to which the external force is applied is moved downwards, the first mounting plate 3 pulls the synchronous pedal link 7 to rotate about the synchronous shaft 8, meanwhile, the synchronous shaft 8 is rotated, and the synchronous shaft in turn drives the synchronous pedal link 7 on the other side to rotate; the synchronous pedal link 7 on the other side in turn drives the first mounting plate 3 on the other side to move downwards, and then the synchronous pedal 2 on the other side is lowered, realizing uniformity of the synchronous pedal assemblies on the two sides.

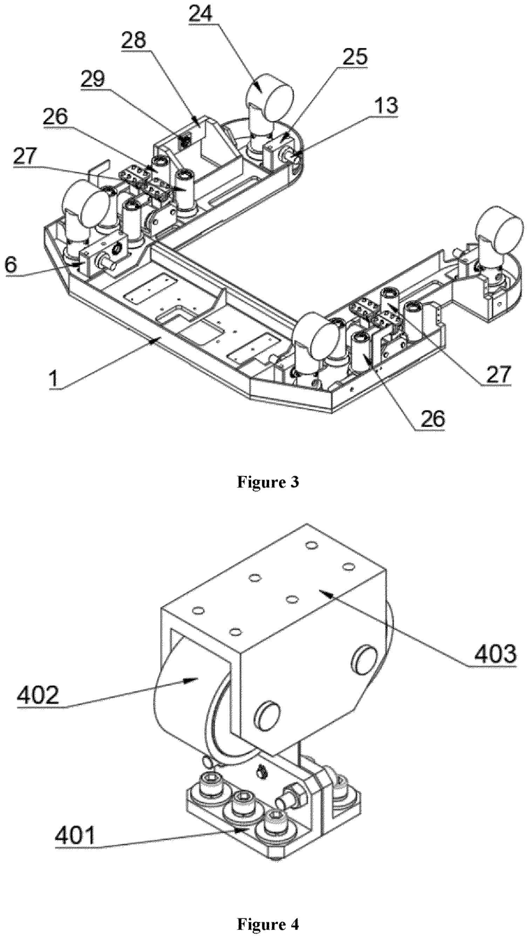

As shown in Figure 2 and Figure 3, first guide mechanisms are arranged at two sides of the first balance assembly 4. The first guide mechanism includes a moving component and a guide component. One of the moving component and the guide component is arranged on the U-shaped base, and the other is arranged on the synchronous pedal assembly. The moving component and the guide component cooperate to provide guidance for movement of the synchronous pedal assembly.

In this embodiment, the moving component is a first guide shaft 5, and the guide component is a first guide sleeve 26. The first guide shaft 5 is arranged on the first mounting plate 3, and the first guide sleeve 26 is arranged corresponding to the first guide shaft 5 under the U-shaped base 1. The first guide shaft 5 slides in the first guide sleeve 26 to realize guidance.

In other embodiments, the first guide shaft 5 may be arranged under the U-shaped base 1, and the first guide sleeve 26 is arranged on the first mounting plate 3. The first guide shaft 5 slides in the first guide sleeve 26 to achieve guidance.

In other embodiments, the moving component may be a slide block, and the guide component is a guide rail. The slide block is arranged on the first mounting plate 3, and the guide rail is arranged under the U-shaped base 1. The guide rail and the slide block cooperate to achieve guidance.

In other embodiments, the slide block may be arranged under the U-shaped base and the guide rail is arranged on the first mounting plate 3. The guide rail and the slide block cooperate to achieve guidance.

As shown in Figure 6, a proximity switch 9 is disposed on the first mounting plate 3. The proximity switch 9 is arranged close to the synchronous pedal link 7. As shown in Figure 3, a synchronous pedal sensing plate 30 is arranged under the U-shaped base 1. The proximity switch 9 moves up or down with the first mounting plate 3, the synchronous pedal sensing plate 30 determines the position of the synchronous pedal 2 by sensing the position of the proximity switch 9, which allows to know the position of the synchronous pedal 2 in real time. The position of the synchronous pedal 2 may be displayed on a display screen of the doctor-controlled platform.

In other embodiments, the positions of the proximity switch 9 and the synchronous pedal sensing plate 30 may be interchanged, that is, the proximity switch 9 is arranged under the U-shaped base 1, and the synchronous pedal sensing plate 30 is arranged on the first mounting plate 3. The positions of the proximity switch and the synchronous pedal sensing plate can be determined according to actual needs.

In other embodiments, the proximity switch 9 may be arranged on one side of the pedal plate 201, and the synchronous pedal sensing plate 30 is arranged on the U-shaped base 1. Alternatively, the proximity switch 9 is arranged on the first mounting plate 3, and the synchronous pedal sensing plate 30 is arranged on a fixed mounting seat 403. The monitoring of the position of the synchronous pedal 2 can be achieved as long as one of the proximity switch 9 and the synchronous pedal sensing plate 30 is arranged on the U-shaped base 1 with constant relative position or on a non-deformed component fixedly connected to the U-shaped base 1, and the other one of the proximity switch 9 and the synchronous pedal sensing plate 30 is arranged on a component having the same motion trajectory as the synchronous pedal 2.

As shown in Figure 4, the first balance assembly 4 includes a mobile mounting seat 401, an elastic component 402 and a fixed mounting seat 403. The fixed mounting seat 403 is fixedly connected to the U-shaped base 1 under the U-shaped base 1, and the mobile mounting seat 401 is arranged on the first mounting plate 3. One end of the elastic component 402 is connected with the fixed mounting seat 403, and the other end of the elastic component 402 is connected with the mobile mounting seat 401. The structure of the second balance assembly 17 is substantially the same as the structure of the first balance assembly 4. The elasticity of the second balance assembly 17 and the elasticity of the first balance assembly 4 may be the same or different.

The elastic component 402 may be a spring, a bellow, a diaphragm or other elastic component having a certain resilience. In this embodiment, the elastic component is a spring.

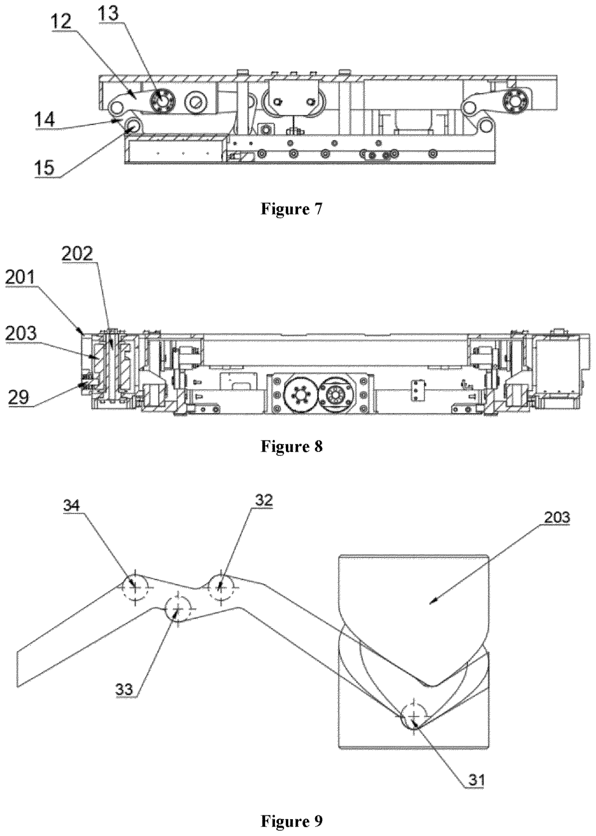

As shown in Figure 8, the pedal plate 201 is a hollow square piece. A rotation shaft 202 is arranged in the pedal plate 201. A limit groove sleeve (that is, a sleeve provided with a limit groove) 203 is sleeved around the rotation shaft 202. The limit groove sleeve 203 is mounted around the rotation shaft 202 through a bearing, and thus the limit groove sleeve 203 is rotatable around the shaft 202. A limit groove is arranged on an outer wall of the limit groove sleeve 203. A distance between the limit groove and a bottom surface of the pedal plate 201 is increased or decreased with the rotation of the limit groove sleeve 203. As shown in Figure 3, a limit shaft fixing seat 28 is arranged at an outer side of the synchronous pedal 2 and is arranged on an outer wall of the U-shaped base 1. A limiting shaft 29 is disposed on the limiting shaft fixing seat 28, and the limiting shaft 29 extends towards the synchronous pedal 2. The limit shaft 29 is inserted into the limit groove. The limit shaft 29 is arranged on the limit shaft fixing seat 28 through a bearing, such that the limit shaft 29 is rotatable freely, thereby reducing friction of the limit shaft 29 when moving in the limit groove. Figure 9 is a schematic deployment diagram of the limit groove. The limit shaft is inserted into the limit groove, and thus the limit shaft can only move along the limit groove. Therefore, when the synchronous pedal 2 of the synchronous pedal assembly and the limit groove sleeve 203 fixed inside the synchronous pedal moves up or down under action of an external force, the limit groove sleeve 203 rotates around the rotation shaft 202 while moving up or down under the restriction of the limit shaft 29. Thus, the limit shaft 29 always moves along the limit groove. It may be considered that the limit shaft 29 is caused, under combination of the up-down movement and the rotation movement of the limit groove sleeve 203, to move in both up-down direction and left-right direction in the deployment diagram of Figure 9. In addition, since the synchronous pedal assembly is always subjected to an upward pulling force of the first balance assembly 4, the limit groove sleeve 203 mounted in the synchronous pedal 2 is also subjected to an upward pulling force. The limit shaft 29 may stop at four position points when moving in the limit groove. When the limit shaft 29 is located at a high stable stop point 31 at the far right of Figure 9, the lower side of the limit shaft 29 abuts against the limit groove under the upward pulling force of the first balance assembly 4 if there is no external force. At this time, the synchronous pedal 2 is at the high stable stop point 31. When the synchronous pedal 2 is pressed down by an external force, the limit groove sleeve 203 is moved downwards, and the limit shaft 29 can only move to the left along the limit groove under restriction of the upper side of the limit groove to the second point from the right, that is, the high-low transition unstable stop point 32. At this time, the synchronous pedal 2 is located at a lowest position. However, at this time, if the external force is withdrawn, the synchronous pedal 2 will cause the limit shaft 29 to move towards the left along the limit groove under combined action of the upward pulling force and the lower side of the limit groove, and finally the limit shaft reaches a stable state at the third point from the right, that is, a low stable stop point 33. At this time, the synchronous pedal 2 is at the lowest position where it can stop steadily. When the synchronous pedal 2 is stepped down again by an external force, the limit shaft 29 can only move to the left along the limit groove again under restriction of the upper side of the limit groove, and reach the fourth point from the right, that is, a low-high transition unstable stop point 34. At this time, if the external force is withdrawn, the synchronous pedal 2 will make the limit shaft 29 move to the left along the limit groove again under the combined action of the upward pulling force and the lower side of the limit groove, and the limit shaft returns to the position of the first point from the right, that is, the high stable stop point 31. In this way, a motion cycle is completed. Therefore, for the synchronous pedal 2 at the high stable stop point, it will reach the lowest but unstable position (the high-low transition unstable stop point 32) after being pressed down, and will stop steadily at the low stable stop point 33 after being released, meanwhile, the control pedal assembly will fall to the ground, which improves the usage comfort. Then, the synchronous pedal 2 will reach the lowest but unstable position (the low-high transition unstable stop point 34) again after being pressed down, and will stop steadily at the high stable stop point 31 after being released, that is, returning to the initial state, at this time, the control pedal assembly is stopped at a height, which ensures passability of the doctor-controlled platform when being moved.

As shown in Figure 5, the control pedal support 10 includes a main support 1001, link receiving plates 1002 and second mounting plates 1003. The main support 1001 is located under the rear side of the U-shaped base 1. The link receiving plates 1002 are vertically disposed on two sides of the main support 1001, and the second mounting plates 1003 are horizontally arranged at outer sides of the link receiving plates 1002. The synchronous pedal-hanging pillar 11 is disposed on the second mounting plate 1003. Convex lugs are provided at opposite ends of each link receiving plate 1002. The convex lugs at the opposite ends are hingedly connected with hinged links 14 through hinged shafts 15. The hinged links 14 are hingedly connected with the brake links 12. The brake links 12 (two brake links located under the rear side of the U-shaped base 1) near the synchronous shaft 8 are fixed on the rear brake synchronous shaft seats 6. As shown in Figure 2, two sides of the U-shaped base 1 away from the synchronous shaft 8 are provided with front brake shaft seats 25, and the brake links 12 (two brake links located at the front side of the U-shaped base 1) away from the synchronous shaft 8 are fixed on the front brake shaft seats 25. Casters 24 are arranged under the U-shaped base 1 close to the rear brake synchronous shaft seat 6, and are also arranged under the U-shaped base 1 close to the front brake shaft seat 25. In total, four casters 24 are arranged under the U-shaped base 1. The four casters 24 constitute a quadrilateral to support the doctor-controlled platform. The four casters 24 are casters with central control brake. As shown in Figure 7, the brake link 12 is connected with the rear brake synchronous shaft seat 6 or the front brake shaft seat 25 through the brake shaft 13, the brake link 12 is locked with the brake shaft 13 through an expansion sleeve, and rotation of the brake link 12 drives the brake shaft 13 to rotate, and rotation of the brake shaft 13 can lock or loosen the caster 24. When the control pedal assembly moves up or down with the synchronous pedal assembly, the link receiving plate 1002 causes the brake link 12 to swing, thereby driving the brake shaft 13 to rotate, and rotation of the brake shaft 13 can control the locking or loosening of the central control casters. In this way, up or down of the control pedal assembly and locking or loosening of the central control casters can be realized under action of a single external force.

An adjustment part is provided in the castor with central control brake, and a hexagonal hole is disposed in the adjustment part. An end of the brake shaft has a hexagonal shape. The hexagonal shape end of the brake shaft is inserted into the adjustment part, and thus rotation of the brake shaft drives the adjustment part to rotate. The rotation of the adjustment part realizes locking or loosening of the caster. The specific structure of the castor with central control brake is available from the prior art and reference may be made to patent CN204845381 U, which is not described in detail herein.

In other embodiments, the four casters may include two casters with central control brake and two directional wheels. The two casters with central control brake are arranged under the open end of the U-shaped base or under the closed end of the U-shaped base, and the two directional wheels are arranged under the other end of the U-shaped base. Correspondingly, as for the directional wheels, there is no need to provide corresponding brake shaft seat, brake shaft or brake link.

In other embodiments, in the case that all of the four casters are casters with central control brake and if braking force provided by two of the brake casters is sufficient to stop the doctor-controlled platform stably, the front brake shaft seat and the corresponding brake shaft and brake link may be removed, or the rear brake synchronous shaft seat and the corresponding brake shaft and brake link may be removed.

As shown in Figure 5, guide rails are disposed on two sides of the control pedal mounting seat 16. Slide blocks are arranged on one side of the link receiving plate 1002. The control pedal mounting seat 16 is slidably connected with the link receiving plates 1002 through the guide rails 23 and the slide blocks. Users can adjust the position of the control pedal mounting seat 16 according to their own needs, therefore, it is suitable for different users, and thus the usage comfort is improved.

In order to better control the position of the control pedal mounting seat 16, a leading screw fixing sleeve 18 is disposed at the bottom of the control pedal mounting seat 16, a leading screw 19 is arranged in the leading screw fixing sleeve 18, and the other end of the leading screw is provided with a driven gear 20. The driven gear 20, a driving gear 21 and a driving mechanism are fixedly arranged on the control pedal support 1001. The driving mechanism drives the driving gear 21 to rotate. The driving gear 21 matches with the driven gear 20. The driven gear 20 is connected with the leading screw 19 through threads. The main support 1001 is provided therein with a through hole corresponding to the leading screw 19, such that the leading screw 19 is movable in the through hole in a telescopic manner. The driving mechanism drives the driving gear 21 to rotate, which in turn drives the driven gear 20 to rotate. The rotation of the driven gear 20 drives the leading screw 19 to move in a telescopic manner, which then drives the leading screw fixing sleeve 18 to move linearly. The leading screw fixing sleeve 18 then causes the control pedal mounting seat 16 to move linearly, thereby achieving precise control of the position of the control pedal mounting seat 16.

Second guide mechanisms are arranged at two sides of the second balance assembly 17. The second guide mechanism includes a moving component and a guide component. One of the moving component and the guide component is disposed on the U-shaped base 1, and the other of the moving component and the guide component is disposed on the control pedal assembly. The moving component and the guide component cooperate to provide guidance for movement of the control pedal assembly.

In this embodiment, the moving component is a second guide shaft 22, and the guide component is a second guide sleeve 27. The second guide shaft 22 is arranged on the second mounting plate 1003, and the second guide sleeve 27 is arranged corresponding to the second guide shaft 22 under the U-shaped base 1. The second guide shaft 22 is slidable in the second guide sleeve 27 to realize guidance.

In other embodiments, the second guide shaft 22 may be arranged under the U-shaped base 1 and the second guide sleeve 27 is arranged on the second mounting plate 1003. The second guide shaft 22 slides in the second guide sleeve 27 to achieve guidance.

In other embodiments, the moving component may be a slide block, and the guide component is a guide rail. The slide block is arranged on the second mounting plate 1003, and the guide rail is arranged under the U-shaped base 1. The guide rail and the slide block cooperate to achieve guidance.

In other embodiments, the slide block may be arranged under the U-shaped base and the guide rail is arranged on the second mounting plate 1003. The guide rail and the slide block cooperate to achieve guidance.

The above specific embodiments are used only to illustrate the technical solutions of the disclosure rather than any restriction. Notwithstanding the detailed description of the disclosure by reference to examples, ordinary persons skilled in the art shall understand that the technical solutions of this disclosure may be modified or equivalently replaced without departing from the scopes of the technical solutions of this disclosure, which shall fall within the scope of the claims of this application.

## Claims

1. A doctor-controlled platform base, **characterized by** comprising a U-shaped base, synchronous pedal assemblies, a control pedal assembly and a brake assembly; the synchronous pedal assemblies are provided at bottoms of two sides of the U-shaped base through first balance assemblies and first guide mechanisms;
the control pedal assembly is arranged in an opening of the U-shaped base, and two sides of the control pedal assembly are connected with a lower surface of the U-shaped base through second balance assemblies and second guide mechanisms, and the two sides of the control pedal assembly are connected with the synchronous pedal assemblies;
a walking device is arranged under the U-shaped base, one end of the brake assembly is connected with the control pedal assembly in a hinged manner and the other end of the brake assembly is connected with the walking device;
two synchronous pedal assemblies move up or down synchronously when an external force is applied to at least one of the synchronous pedal assemblies, which simultaneously drive the control pedal assembly to move up or down in the same direction as the synchronous pedal assemblies; the control pedal assembly, when moving up or down, can drive the brake assembly to move, to realize locking or loosening of the walking device.

2. The doctor-controlled platform base according to claim 1, **characterized in that** the control pedal assembly comprises a control pedal support, a control pedal mounting seat, the second balance assemblies and the second guide mechanisms, the control pedal mounting seat is disposed on the control pedal support, one end of the second balance assembly is connected with the control pedal support, and the other end of the second balance assembly is connected with the U-shaped base; the control pedal support is connected with the synchronous pedal assemblies.

3. The doctor-controlled platform base according to claim 2, **characterized in that** one of the control pedal support and the synchronous pedal assembly is provided with a convex snap fitting feature, and the other of the control pedal support and the synchronous pedal assembly is provided with a concave snap fitting feature capable of matching with the convex snap fitting feature.

4. The doctor-controlled platform base according to claim 2, **characterized in that** the control pedal support is connected with the synchronous pedal assemblies in a hinged manner or in a snap fit manner, and in case that they are connected in a hinged manner, the control pedal support is provided thereon with hinged bars, and the synchronous pedal assemblies are hingedly connected with the hinged bars.

5. The doctor-controlled platform base according to any one of claims 2-4, **characterized in that** one side of the control pedal mounting seat close to a closed end of the U-shaped base is connected with the control pedal support through a telescopic device.

6. The doctor-controlled platform base according to claim 5, **characterized in that** the telescopic device comprises a leading screw fixing sleeve, a leading screw, a driven gear, a driving gear and a driving mechanism, and wherein the leading screw fixing sleeve is arranged at a bottom of the control pedal mounting seat, the leading screw is arranged in the leading screw fixing sleeve, the driven gear is arranged on the other end of the leading screw, and the driven gear is engaged with the driving gear; the driven gear and the driving gear are arranged on the control pedal support, and the driving mechanism drives the driving gear to rotate; the driven gear is connected with the leading screw through threads, and the control pedal support is provided therein with a through hole corresponding to the leading screw, and the leading screw is movable in the through hole in a telescopic manner.

7. The doctor-controlled platform base according to claim 1, **characterized in that** the synchronous pedal assemblies comprise two synchronous pedals, two first mounting plates, two first balance assemblies and two first guide mechanisms; the two first mounting plates are arranged on two sides of the U-shaped base, respectively, and the synchronous pedals and the first balance assemblies are arranged on the first mounting plates; the first balance assemblies are fixed under the U-shaped base, and the synchronous pedals are connected with the control pedal assembly.

8. The doctor-controlled platform base according to claim 7, **characterized in that** the first balance assembly comprises a mobile mounting seat, an elastic component and a fixed mounting seat, the fixed mounting seat is fixed under the U-shaped base, and the mobile mounting seat is arranged on the first mounting plate; one end of the elastic component is connected with the fixed mounting seat, and the other end of the elastic component is connected with the mobile mounting seat.

9. The doctor-controlled platform base according to claim 7 or 8, **characterized in that** the synchronous pedal comprises a pedal plate provided with a limit structure, and a limit shaft is mounted on the U-shaped base; the limit shaft is inserted in the limit structure; the limit structure has at least an upper limit part and a lower limit part; the limit structure is capable of coordinating with the upper limit part or the lower limit part to achieve positioning when the synchronous pedal assembly moves up or down to a preset position.

10. The doctor-controlled platform base according to claim 9, **characterized in that** the limit structure comprises a rotation shaft and a limit groove sleeve, the rotation shaft is inserted in the limit groove sleeve, a limit groove is disposed in an outer wall of the limit groove sleeve, and a distance between the limit groove and a bottom surface of the pedal plate increases or decreases with rotation of the limit groove sleeve; and the limit shaft is inserted in the limit groove.

11. The doctor-controlled platform base according to claim 7, **characterized by** further comprising a sensing device for determining position of the synchronous pedal; the sensing device comprises a sensing plate and a proximity switch; the sensing plate or the proximity switch is disposed on the U-shaped base.

12. The doctor-controlled platform base according to claim 1, **characterized in that** the walking device comprises at least two casters with central control brake; the brake assembly comprises at least two brake shaft seats, brake shafts and brake links;
the brake shaft seats are arranged under two sides of an open end of the U-shaped base and are close to the casters with central control brake, one end of each brake link is hingedly connected with the control pedal assembly, and the other end of each brake link is connected with one caster with central control brake through corresponding brake shaft;
each brake shaft is inserted in a corresponding brake shaft seat, and is rotatable in the corresponding brake shaft seat, to realize locking or loosening of a driving wheel.

13. The doctor-controlled platform base according to claim 1, **characterized in that** the first guide mechanism has the same structure as the second guide mechanism.
